# EUROPEAN PATENT APPLICATION

(11) **EP 4 810 009 A1**
(43) Date of publication of application: **23.09.2026**
(21) Application number: 24891451.7
(22) Date of filing: 14.11.2024
(51) Int. Cl.: A61K 8/365, A61Q 19/00, A61Q 19/02

(54) **MELANOGENESIS INHIBITOR AND EXTERNAL PREPARATION FOR SKIN CONTAINING SAME**

(30) Priority: 17.11.2023 JP 2023195826; 04.10.2024 JP 2024175023
(71) Applicant: MITSUBISHI GAS CHEMICAL COMPANY, INC., Chiyoda-ku Tokyo 100-8324 (JP)
(72) Inventor: OKADA Takumi, Niigata-shi, Niigata 950-3112 (JP); IKEMOTO Kazuto, Niigata-shi, Niigata 950-3112 (JP)
(74) Representative: Vossius & Partner Patentanwälte Rechtsanwälte mbB
(86) International application number: PCT/JP2024/040413
(87) International publication number: WO 2025/105415

(57) **Abstract**

According to one embodiment, provided is a melanogenesis inhibitor, comprising a compound represented by the following formula (I) or a salt thereof: 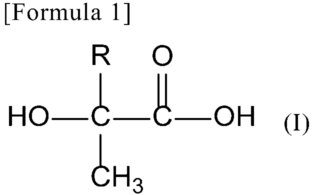 wherein R represents an alkyl group containing 1 to 4 carbon atoms.

## Description

### Technical Field

The present invention relates to a melanogenesis inhibitor, and an external preparation for skin, comprising the same.

### Background Art

When skin is exposed to ultraviolet rays contained in sunlight, UV lamps and the like, it loses its luster, texture, moisture, etc. In particular, if the dermis is damaged by ultraviolet rays, wrinkles and sagging are generated, thereby causing a condition known as photoaging. Reactive oxygen generated by UV exposure and various factors released from skin cells by the influence of UV exposure accelerate tyrosinase activity in melanocytes. Melanin involved in the color tone of skin is generated by oxidation of tyrosine in melanocytes by tyrosinase. When tyrosinase is activated by ultraviolet rays, melanin is excessively produced, which is then passed to epidermal cells, so that it is considered that the color tone of skin is changed and darkened.

Therefore, it has been known that suppression of melanogenesis is effective for achieving a whitening effect. Known active ingredients for suppressing melanogenesis include ascorbic acid, kojic acid, arbutin, ellagic acid, 4-alkylresorcinol, or their derivatives, and various plant extracts. Moreover, other than these, it has been known that the combined use of a compound consisting of menthol and an ester of a long-chain unsaturated fatty acid containing 18 or more carbon atoms, with an inhibitor of a signaling substance produced from keratinocytes, an antioxidant, an anti-inflammatory agent, a high-molecular-weight compound and a polyhydric alcohol, exhibits a higher synergistic melanogenesis-inhibiting effect (for example, Patent Literature 1). Furthermore, it has also been known that cedrol, a component of sandalwood oil, exhibits a melanogenesis-inhibiting effect (for example, Patent Literature 2), and that nerolidol, a component of cabreuva oil, exhibits a melanogenesis-inhibiting effect (for example, Patent Literature 3).

Meanwhile, Vetiver extract is known to exhibit a melanogenesis-promoting effect (for example, Patent Literature 4).

Thus, while various compounds are known to be involved in melanogenesis, in recent years, the harmful effects of ultraviolet rays have become widely recognized, and melanogenesis inhibitors have been attracting increasing attention.

### Citation List

### Patent Literature

Patent Literature 1: Patent Publication (Kokai) No. 2007-161591 A
Patent Literature 2: Patent Publication (Kokai) No. 10-36246 A (1998)
Patent Literature 3: Patent Publication (Kokai) No. 6-72855 A (1994)
Patent Literature 4: Patent Publication (Kokai) No. 2011-157317 A

### Summary of Invention

### Technical Problem

It is an object of the present invention to provide a melanogenesis inhibitor, and an external preparation for skin, comprising the same.

### Solution to Problem

The present inventors have conducted intensive studies directed towards achieving the above-described object, and as a result, the inventors have found that a compound having a specific structure or a salt thereof has a melanogenesis-inhibiting effect, thereby completing the present invention. The present invention is, for example, as follows.
[1] A melanogenesis inhibitor, comprising a compound represented by the following formula (I) or a salt thereof: 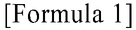 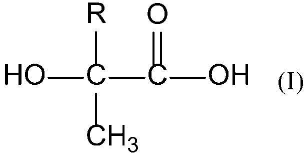 wherein R represents an alkyl group containing 1 to 4 carbon atoms.
[2] The melanogenesis inhibitor according to the above [1], wherein R in the formula (I) is a methyl group.
[3] The melanogenesis inhibitor according to the above [1] or [2], which further has any one or more of the following effects (a) to (d):
   (a) solubility in oils,
   (b) solubility in oils and fats,
   (c) chemical peeling ability, and
   (d) ability to eliminate acne bacteria.
[4] An external preparation for skin, comprising the melanogenesis inhibitor according to any one of the above [1 to 3.
[5] The external preparation for skin according to the above [4], which comprises the compound represented by the formula (I) or a salt thereof in an amount of 0.001% to 50% by weight with respect to the external preparation for skin.
[6] The external preparation for skin according to the above [4] or [5], which further comprises one or more additives selected from the group consisting of a thickener, a pH adjuster, an antiseptic, and an antioxidant.
[7] The external preparation for skin according to the above [6], which comprises the additive(s) in each amount of 0.01% to 20% by weight each.
[8] The external preparation for skin according to any one of the above [4] to [7], which has a pH value of 0.5 to 12.
[9] The external preparation for skin according to any one of the above [4] to [8], which is a whitening cosmetic.
[10] The external preparation for skin according to the above [9], wherein the whitening cosmetic is selected from the group consisting of the group consisting of a lotion, a cream, an emulsion, a gel, a serum, a facial cleanser, a soap, an ointment, a pack agent, and a foundation.

### Advantageous Effects of Invention

According to the present invention, a melanogenesis inhibitor, and an external preparation for skin, comprising the same, can be provided.

### Brief Description of Drawings

[Figure 1] Figure 1 is a view showing the results of Example 1.
[Figure 2] Figure 2 is a view showing the results of Example 2.
[Figure 3] Figure 3 is a view showing the results of Example 3.
[Figure 4] Figure 4 is a view showing the results of Example 4.
[Figure 5] Figure 5 is a view showing the results of Example 5.

### Description of Embodiments

Hereinafter, the embodiments of the present invention will be described in detail.

### Melanogenesis inhibitor

[1] According to one embodiment of the present invention, a melanogenesis inhibitor, comprising a compound represented by the following formula (I) or a salt thereof, is provided: 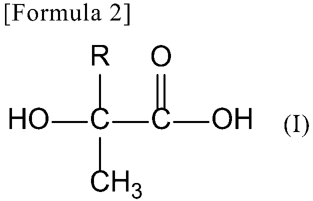 wherein R represents an alkyl group containing 1 to 4 carbon atoms.

The alkyl group containing 1 to 4 carbon atoms may be linear or branched, and examples thereof may include methyl, ethyl, propyl, isopropyl, n-butyl, isobutyl, and t-butyl. R is preferably methyl, ethyl, or isopropyl, more preferably methyl or ethyl, and particularly preferably methyl. When R is methyl, the compound of the formula (I) is 2-hydroxyisobutyric acid, and this compound is hereinafter also referred to as "methyl lactic acid."

The salt of compound of the formula (I) is not limited, and a pharmaceutically acceptable salt can be used. Examples of such salt may include sodium salt, potassium salt, magnesium salt, and calcium salt. Of these, sodium salt and potassium salt are preferable.

The melanogenesis inhibitor according to the embodiment can effectively suppress melanogenesis. In addition, an external preparation for skin, comprising the present melanogenesis inhibitor is stable, highly safe, and has an excellent whitening effect. The reason that such an effect is obtained is unknown, but is assumed as follows.

Melanin is biosynthesized in melanosomes, membrane organelles within pigment cells. It is assumed that melanogenesis can be suppressed by regulating the expression and activity of tyrosinase, which is thought to be a rate-limiting enzyme in melanin biosynthesis, by the compound of the formula (I). It is assumed that the reaction of converting tyrosine to dopaquinone and dopa is suppressed by regulating the expression and activity of tyrosinase, and as a result, melanogenesis is suppressed.

As described in Examples later, the compound of the above formula (1) had a tyrosinase biosynthesis inhibitory action and a tyrosinase protein expression inhibitory action, and further had a melanin synthesis-related factor mRNA expression-suppressing action. Therefore, according to one embodiment, a tyrosinase biosynthesis inhibitor, comprising the compound represented by the above formula (1) or a salt thereof, is provided. Moreover, according to another embodiment, a tyrosinase protein expression inhibitor, comprising the compound represented by the above formula (1) or a salt thereof, is provided. According to a further embodiment, a melanin synthesis-related factor mRNA expression-suppressing agent, comprising the compound represented by the above formula (1) or a salt thereof, is provided.

The melanogenesis inhibitor according to the embodiment can be used in combination with known other melanogenesis inhibitors (for example, pantetheine-s-sulfonate, isoferulic acid, ascorbic acid and their derivatives, hydroquinone and its derivatives, arbutin, kojic acid, linoleic acid (ester), ellagic acid, glycyrrhizic acid, lactic acid (ester), 4-alkylresorcinol, licorice extract, placenta extract, etc.). When multiple types of melanogenesis inhibitors are used in combination, they may be used simultaneously, sequentially, or separately. As described below, multiple types of melanogenesis inhibitors may also be mixed into a single external preparation for skin.

### [2] External preparation for skin

According to another embodiment of the present invention, an external preparation for skin, comprising the aforementioned melanogenesis inhibitor, is provided.

The compound represented by the above formula (I) or a salt thereof is comprised in the external preparation for skin, in an amount of, for example, 0.001% to 50% by weight, 0.001 to 5% by weight, or 0.01 to 0.1% by weight. By allowing the external preparation for skin to comprise the compound of the formula (I) or a salt thereof in such an amount, a favorable melanogenesis-inhibiting effect can be obtained. The content of the compound of the formula (I) or a salt thereof can be appropriately set, depending on the type and intended use of the external preparation for skin.

Utilizing such a melanogenesis-inhibiting effect, the aforementioned external preparation for skin can be used as a whitening cosmetic. The whitening cosmetic is not particularly limited, as long as it is applied to the skin. Examples of the whitening cosmetic may include lotions, a cream, an emulsion, a gel, a serum, a facial cleanser, a soap, an ointment, a pack agent, and a foundation. Such a whitening cosmetic can be produced according to commonly used formulation methods.

In addition to the melanogenesis inhibitor according to the embodiment, the external preparation for skin may comprise additives commonly used in cosmetics, pharmaceutical products, etc. Examples of such additives may include a powder component, liquid oil and fat, solid oil and fat, waxes, hydrocarbon oil, higher fatty acid, higher alcohol, synthetic ester oil, silicone, an anionic surfactant, a cationic surfactant, an amphoteric surfactant, a nonionic surfactant, a moisturizer, a water-soluble polymeric compound, a thickener, a coating agent, an ultraviolet absorber, an ultraviolet blocker, an antiseptic, a sequestering agent, lower alcohol, polyhydric alcohol, sugars, an amino acid derivative, organic amines, a synthetic resin emulsion, a pH adjuster, a skin nutrient, vitamins, an antioxidant, an antioxidant aid, a fragrance, and water. Among these additives, some additives can be selected as needed, and can be appropriately blended. Among others, the external preparation for skin according to the embodiment preferably comprises one or more additives selected from the group consisting of a thickener, a pH adjuster, an antiseptic, and an antioxidant.

Furthermore, vitamins, a skin activator, a blood circulation promoter, a resident flora control agent, an active oxygen scavenger, an anti-inflammatory agent, other whitening agents, other medicinal components such as a bactericide, and physiological components can be appropriately mixed into the external preparation for skin, as needed.

The aforementioned additives may be used alone as a single type, or may also be used in combination of multiple types. The amount of additives used is not particularly limited, and for example, the additives is added in each amount (per each additive) of 0.01% to 20% by weight, 0.01% to 5% by weight, or 0.01% to 1% by weight, with respect to the external preparation for skin. Moreover, the additives are preferably added in a total amount of 0.1% to 50% by weight, 0.1% to 20% by weight, or 0.1% to 5% by weight, with respect to the external preparation for skin. By allowing the external preparation for skin to comprise additives in these amounts, the effects of the compound of formula (I) or a salt thereof, which serves as an active ingredient, are not impaired, and also, the effects of the additives are also sufficiently exerted.

Hereinafter, specific examples of additives are listed.

Examples of the powder component may include: talc, kaolin, mica, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (calcined gypsum), calcium phosphate, hydroxyapatite, ceramic powder, metal soaps (zinc myristate, calcium palmitate, and aluminum stearate), polyamide resin powder (nylon powder), polyethylene powder, polymethyl methacrylate powder, polystyrene powder, styrene-acrylic acid copolymer resin powder, organic powders such as cellulose powder, and inorganic white pigments such as titanium dioxide and zinc oxide; inorganic red pigments such as iron oxide (red iron oxide) and iron titanate; inorganic purple pigments such as carbon black, mango violet and cobalt violet; inorganic green pigments such as cobalt titanate; inorganic blue pigments such as ultramarine and Prussian blue; pearl pigments such as titanium oxide-coated mica, titanium oxide-coated bismuth oxychloride, titanium oxide-coated talc, colored titanium oxide-coated mica, bismuth oxychloride, and fish scale foil; metal powder pigments such as aluminum powder and copper powder; organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; organic pigments including zirconium, barium, or aluminum lakes, such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3 and Blue No. 1; natural pigments such as chlorophyll and beta-carotene. However, these powder components are not particularly limited as long as they are powders that can be applied to general cosmetic products, and are not limited to the above-described components.

Examples of the liquid oil and fat may include avocado oil, camellia oil, evening primrose oil, turtle oil, macadamia nut oil, corn oil, mink oil, olive oil, rapeseed oil, egg yolk oil, sesame oil, wheat germ oil, castor oil, linseed oil, safflower oil, cottonseed oil, soybean oil, peanut oil, tea seed oil, rice bran oil, jojoba oil, germ oil, triglyceride, glyceryl trioctanoate, and glyceryl triisopalmitate.

Examples of the solid oil and fat may include cocoa butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, beef foot fat, Japan wax, and hydrogenated castor oil.

Examples of the waxes may include beeswax, candelilla wax, cotton wax, rice bran wax, carnauba wax, bayberry wax, Chinese wax, spermaceti wax, lanolin, lanolin acetate, liquid lanolin, lanolin fatty acid isopropyl, hexyl laurate, reduced lanolin, jojoba wax, hard lanolin, POE (polyoxyethylene) lanolin alcohol ether, POE lanolin alcohol acetate, POE cholesterol ether, lanolin fatty acid polyethylene glycol, and POE hydrogenated lanolin alcohol ether.

Examples of the hydrocarbon oil may include liquid paraffin, squalene, paraffin, squalane, petrolatum, and microcrystalline wax.

Examples of the higher fatty acid may include lauric acid, myristic acid, palmitic acid, stearic acid, oleic acid, 12-hydroxystearic acid, undecylenic acid, tall oil acid, isostearic acid, linoleic acid, linolenic acid, eicosapentaenoic acid (EPA), and docosahexaenoic acid (DHA).

Examples of the higher alcohol may include: straight-chain alcohols such as lauryl alcohol, cetyl alcohol, stearyl alcohol, behenyl alcohol, myristyl alcohol, oleyl alcohol, and cetostearyl alcohol; and branched-chain alcohols such as monostearyl glycerin ether, 2-decyltetradecinol, lanolin alcohol, cholesterol, phytosterol, hexyldodecanol, isostearyl alcohol, and octyldodecanol.

Examples of the synthetic ester oil may include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, and isocetyl stearate,. isocetyl isostearate, cholesteryl 12-hydroxystearate, ethylene glycol di-2-ethylhexylate, dipentaerythritol fatty acid ester, neopentyl glycol dicaprate, diisostearyl malate, glycerin di-2-heptylundecanoate, trimethylolpropane tri-2-ethylhexylate, trimethylolpropane triisostearate, pentaneerythritol tetra-2-ethylhexylate, glycerin tri-2-ethylhexylate, trimethylolpropane triisostearate, cetyl 2-ethylhexanoate, 2-ethylhexyl palmitate, glycerin trimyristate, tri-2-heptylundecanoic acid glyceride, castor oil fatty acid methyl ester, oleic acid oil, cetostearyl alcohol, 2-heptylundecyl palmitate, diisobutyl adipate, N-lauroyl-L-glutamic acid 2-octyldodecyl ester, di-2-heptylundecyl adipate, ethyl laurate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, and triethyl citrate.

Examples of the silicone may include: linear polysiloxanes such as dimethylpolysiloxane, methylphenylpolysiloxane, and methylhydrogenpolysiloxane; alicyclic polysiloxanes such as decamethylpolysiloxane, dodecamethylpolysiloxane, and tetramethyltetrahydrogenpolysiloxane; and silicone resins and silicone rubbers that form three-dimensional network structures.

Examples of the anionic surfactant may include: fatty acid soaps such as soap base, sodium laurate and sodium palmitate; higher alkyl sulfates such as sodium lauryl sulfate and potassium lauryl sulfate; alkyl ether sulfates such as POE triethanolamine lauryl sulfate and POE sodium lauryl sulfate; N-acyl sarcosinates such as sodium lauroyl sarcosinate; higher fatty acid amide sulfonates such as sodium N-myristoyl-N-methyl taurate, sodium cocoyl methyl tauride, and sodium lauryl methyl tauride; phosphate ester salts such as sodium POE oleyl ether phosphate and POE stearyl ether phosphate; sulfosuccinates such as sodium di-2-ethylhexyl sulfosuccinate, sodium monolauroyl monoethanolamido polyoxyethylene sulfosuccinate, and sodium lauryl polypropylene glycol sulfosuccinate; alkylbenzenesulfonates such as sodium linear dodecyl benzene sulfonate, triethanolamine linear dodecyl benzene sulfonate, and linear dodecylbenzenesulfonic acid; N-acylglutamates such as monosodium N-lauroylglutamate, disodium N-stearoylglutamate, and monosodium N-myristoyl-L-glutamate; higher fatty acid ester sulfates such as sodium hydrogenated coconut oil fatty acid glycerin sulfate; sulfated oils such as Turkey red oil; and POE alkyl ether carboxylic acid, POE alkyl allyl ether carboxylate, α-olefin sulfonate, higher fatty acid ester sulfonate, secondary alcohol sulfate, higher fatty acid alkylolamide sulfate, sodium lauroyl monoethanolamide succinate, N-palmitoyl aspartic acid ditriethanolamine, and sodium caseinate.

Examples of the cationic surfactant may include: alkyltrimethylammonium salts such as stearyltrimethylammonium chloride and lauryltrimethylammonium chloride; alkylpyridinium salts such as distearyldimethylammonium chloride, dialkyldimethylammonium salts, poly(N,N'-dimethyl-3,5-methylenepiperidinium) chloride, and cetylpyridinium chloride; and alkyl quaternary ammonium salts, alkyldimethylbenzylammonium salts, alkylisoquinolinium salts, dialkylmorphonium salts, POE alkylamine, alkylamine salts, a polyamine fatty acid derivative, an amyl alcohol fatty acid derivative, benzalkonium chloride, and benzethonium chloride.

Examples of the amphoteric surfactant may include: imidazoline-based amphoteric surfactants such as 2-undecyl-N,N,N-(hydroxyethylcarboxymethyl)-2-imidazoline sodium and 2-cocoyl-2-imidazolinium hydroxide-1-carboxyethyloxy disodium salt; and betaine-based surfactants such as 2-heptadecyl-N-carboxymethyl-N-hydroxyethylimidazolinium betaine, lauryl dimethylaminoacetic acid betaine, alkyl betaine, amido betaine, and sulfobetaine.

Examples of the lipophilic nonionic surfactant may include: sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; and glycerin polyglycerin fatty acids such as glycerin mono-cottonseed oil fatty acid, glycerin mono-erucate, glycerin sesquioleate, glycerin monostearate, glyceryl α,α'-oleate pyroglutamate and glyceryl monostearate malate, propylene glycol fatty acid esters such as propylene glycol monostearate, a hydrogenated castor oil derivative, and glycerin alkyl ether.

Examples of the hydrophilic nonionic surfactant may include: POE sorbitan fatty acid esters such as POE sorbitan monooleate, POE-sorbitan monostearate, POE-sorbitan monooleate, and POE-sorbitan tetraoleate; POE sorbit fatty acid esters such as POE-sorbit monolaurate, POE-sorbit monooleate, POE-sorbit pentaoleate, and POE-sorbit monostearate; POE glycerin fatty acid esters such as POE-glycerin monostearate, POE-glycerin monoisostearate, and POE-glycerin triisostearate; POE fatty acid esters such as POE monooleate, POE distearate, POE monodioleate, and ethylene glycol cystearate; POE alkyl ethers such as POE lauryl ether, POE oleyl ether, POE stearyl ether, POE behenyl ether, POE 2-octyldodecyl ether, and POE cholestanol ether; POE alkyl phenyl ethers such as POE octylphenyl ether, POE nonylphenyl ether, and POE dinonylphenyl ether; Pluronics such as Pluronic; POE-POP alkyl ethers such as POE-POP cetyl ether, POE-POP2-decyltetradecyl ether, POE-POP monobutyl ether, POE-POP hydrogenated lanolin, and POE-POP glycerin ether; tetraPOE-tetraPOP ethylenediamine condensates such as Tetronic; POE hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearate diester, and POE hydrogenated castor oil maleic acid; POE beeswax and lanolin derivatives such as POE sorbit beeswax; alkanolamides such as coconut oil fatty acid diethanolamide, lauric acid monoethanolamide, and fatty acid isopropanolamide; and POE propylene glycol fatty acid ester, POE alkylamine, POE fatty acid amide, sucrose fatty acid ester, a POE nonylphenyl formaldehyde condensate, alkylethoxydimethylamine oxide, and trioleyl phosphate.

Examples of the moisturizer may include polyethylene glycol, propylene glycol, glycerin, 1,3-butylene glycol, xylitol, sorbitol, maltitol, chondroitin sulfate, hyaluronic acid, mucoitin sulfate, cholesteryl-12-hydroxystearate, ceramide, glucosylceramide, sodium lactate, bile acid salts, dl-pyrrolidone carboxylate, short-chain soluble collagen, diglycerin (EO) PO adduct, Chestnut rose extract, yarrow extract, and melilot extract.

Examples of the natural water-soluble polymeric compound may include: plant-based polymeric compounds such as gum Arabic, tragacanth gum, galactan, carob gum, karaya gum, carrageenan, pectin, agar, quince seed (quince), algae colloid (brown algae extract), starch (rice, corn, potato, and wheat), and glycyrrhizic acid; microorganism-based polymeric compounds such as xanthan gum, dextran, succinoglucan, and pullulan; and animal-based polymeric compounds such as collagen, casein, albumin, and gelatin.

Examples of the semisynthetic water-soluble polymeric compound may include: starch-based polymeric compounds such as carboxymethyl starch and methylhydroxypropyl starch; cellulose-based polymeric compounds such as methylcellulose, nitrocellulose, ethylcellulose, methylhydroxypropyl cellulose, hydroxyethyl cellulose, sodium cellulose sulfate, hydroxypropyl cellulose, sodium carboxymethylcellulose (CMC), crystalline cellulose, and cellulose powder; and alginic acid-based polymeric compounds such as sodium alginate and propylene glycol ester alginate.

Examples of the synthetic water-soluble polymeric compound may include: vinyl-based polymeric compounds such as polyvinyl alcohol, polyvinyl methyl ether, polyvinylpyrrolidone, and carboxyvinyl polymers (e.g., "Carbopol^{®}" manufactured by Lubrizol Advanced Materials); polyoxyethylene-based polymeric compounds such as polyethylene glycol 20,000, 4,000,000, and 600,000; polyoxyethylene-polyoxypropylene copolymer copolymeric high-molecular-weight compounds; acrylic-based polymeric compounds such as sodium polyacrylate, polyethyl acrylate, and polyacrylamide; and polyethyleneimine and cationic polymers.

Examples of the inorganic water-soluble polymeric compound may include bentonite, magnesium aluminum silicate (e.g., "VEEGUM" manufactured by Vanderbilt), laponite, hectorite, and silicic anhydride.

Examples of the thickener may include gum Arabic, carrageenan, karaya gum, tragacanth gum, carob gum, quince seed (quince), casein, dextrin, gelatin, sodium pectinate, sodium alginate, methylcellulose, ethylcellulose, hydroxyethylcellulose, hydroxypropylcellulose, sodium polyacrylate, a carboxyvinyl polymer, dialkyldimethylammonium cellulose sulfate, xanthan gum, magnesium aluminum silicate, and bentonite.

Examples of the ultraviolet absorber may include: benzoic acid-based ultraviolet absorbers such as para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA methyl ester; anthranilic acid-based ultraviolet absorbers such as homomenthyl-N-acetylanthranilate; salicylic acid-based ultraviolet absorbers such as amyl salicylate, menthyl salicylate, homomenthyl salicylate, octyl salicylate, phenyl salicylate, benzyl salicylate, and p-isopropanol phenyl salicylate; cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxycinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glyceryl-mono-2-ethylhexanoyl-di-para-methoxy cinnamate; benzophenone-based UV absorbers such as 2,4-dihydroxybenzophenone, 2,2'-dihydroxy-4-methoxybenzophenone, 2,2'-dihydroxy-4,4'-dimethoxybenzophenone. 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; and 3-(4'-methylbenzylidene)-d,1-camphor, 3-benzylidene-d,1-camphor, urocanic acid, urocanic acid ethyl ester, 2-phenyl-5-methylbenzoxazole, 2,2'-hydroxy-5-methylphenylbenzotriazole, and 2-(2'-hydroxy-5'-t-octylphenyl)benzotriazole, 2-(2'-hydroxy-5'-methylphenylbenzotriazole, dibenzalazine, dianisoylmethane, 4-methoxy-4'-t-butyldibenzoylmethane, and 5-(3,3-dimethyl-2-norbornylidene)-3-pentan-2-one.

Examples of the ultraviolet blocker may include titanium oxide, talc, carmine, bentonite, kaolin, and zinc oxide.

Examples of the antiseptic may include methylparaben, ethylparaben, propylparaben, phenoxyethanol, and sodium benzoate.

Examples of the sequestering agent may include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, and edetic acid.

Examples of the lower alcohol may include methanol, ethanol, propanol, isopropanol, isobutyl alcohol, and t-butyl alcohol.

Examples of the polyhydric alcohol may include: dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols such as glycerin, trimethylolpropane, and 1,2,6-hexanetriol; tetrahydric alcohols such as pentaerythritol; pentahydric alcohols such as xylitol; hexahydric alcohols such as sorbitol and mannitol; polyhydric alcohol polymers such as diethylene glycol, dipropylene glycol, triethylene glycol, polypropylene glycol, tetraethylene glycol, diglycerin, polyethylene glycol, triglycerin, tetraglycerin, and polyglycerin; dihydric alcohol alkyl ethers such as ethylene glycol monomethyl ether, and ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether; dihydric alcohol alkyl diethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate; glycerin monoalkyl ethers such as xyl alcohol, selachyl alcohol, and batyl alcohol; sugar alcohols such as sorbitol, maltitol, maltotriose, mannitol, sucrose, erythritol, glucose, fructose, starch hydrolysis sugar, maltose, xylitose, and starch hydrolysis sugar reducing alcohol; and glysolid, tetrahydrofurfuryl alcohol, POE tetrahydrofurfuryl alcohol, POP butyl ether. POP/POE butyl ether, tripolyoxypropylene glycerin ether, POP glycerin ether, POP glycerin ether phosphate, and POP/POE pentaneerythritol ether.

Examples of the monosaccharide may include: trioses such as D-glyceraldehyde and dihydroxyacetone; tetraoses such as D-erythrose, D-erythrulose, D-threose, and erythritol; pentoses such as L-arabinose, D-xylose, L-lyxose, D-arabinose, D-ribose, D-ribulose, D-xylulose, and L-xylulose; hexoses such as D-glucose, D-talose, D-busicose, D-galactose, D-fructose, L-galactose, L-mannose, and D-tagatose; heptoses such as aldoheptose and heprose; octoses such as octulose; deoxysugars such as 2-deoxy-D-ribose, 6-deoxy-L-galactose, and 6-deoxy-L-mannose; amino sugars such as D-glucosamine, D-galactosamine, sialic acid, aminouronic acid, and muramic acid; and uronic acids such as D-glucuronic acid, D-mannuronic acid, L-guluronic acid, D-galacturonic acid, and L-iduronic acid.

Examples of the oligosaccharide may include sucrose, guanthianose, umbelliferose, lactose, planteose, isolychnoses, α,α-trehalose, raffinose, lychnoses, umbilicin, and stachyose verbascoses.

Examples of the polysaccharide may include cellulose, quince seed, chondroitin sulfate, starch, galactan, dermatan sulfate, glycogen, gum Arabic, heparan sulfate, hyaluronic acid, tragacanth gum, chondroitin, Xanthan gum, mucoitin sulfate, guar gum, dextran, and caronic acid.

Examples of the amino acid may include: neutral amino acids such as glycine, alanine, valine, leucine, isoleucine, serine, threonine, tryptophan, cystine, cysteine, methionine, proline, and hydroxyproline; acidic amino acids such as aspartic acid, glutamic acid, asparagine, and glutamine; and basic amino acids such as arginine, histidine, lysine, and hydroxylysine.

Examples of the amino acid derivative may include sodium acyl sarcosinate (sodium lauroyl sarcosinate), acyl glutamate, sodium acyl β-alanine, glutathione, and pyrrolidone carboxylic acid.

Examples of the organic amine may include monoethanolamine, diethanolamine, triethanolamine, morpholine, triisopropanolamine, 2-amino-2-methyl-1,3-propanediol, and 2-amino-2-methyl-1-propanol.

Examples of the synthetic resin emulsion may include an acrylic resin emulsion, a polyethyl acrylate emulsion, an acrylic resin liquid, a polyacrylic alkyl ester emulsion, and a polyvinyl acetate resin emulsion.

Examples of the pH adjuster may include buffer agents such as malic acid-sodium malate, lactic acid-sodium lactate, and citric acid-sodium citrate.

Examples of the vitamins may include: vitamins A such as vitamin A oil and retinol; vitamins B1 such as thiamine; vitamins B2 such as riboflavin; vitamins B6 such as pyridoxine hydrochloride; vitamins C such as L-ascorbic acid, L-ascorbic acid phosphate ester, L-ascorbic acid monopalmitate ester, L-ascorbic acid dipalmitate ester, and L-ascorbic acid-2-glucoside; pantothenic acids such as calcium pantothenate; vitamins D such as vitamin D2 and cholecalciferol; vitamins E such as α-tocopherol, tocopherol acetate, and DL-α-tocopherol nicotinate; and pantothenic acid and a derivative thereof, and biotin.

Examples of the antioxidant may include tocopherols, dibutylhydroxytoluene, butylhydroxyanisole, and gallic acid esters. Examples of the antioxidant aid may include phosphoric acid, citric acid, ascorbic acid, maleic acid, malonic acid, succinic acid, fumaric acid, kephalin, hexametaphosphate, phytic acid, and ethylenediaminetetraacetic acid.

The external preparation for skin according to the embodiment may have any dosage form. The aforementioned melanogenesis inhibitor may be blended with one or more types of the aforementioned additives to produce a product of interest according to an ordinary method, and the product can adopt a dosage form appropriate for the product, such as a solution system, a solubilized system, an emulsion system, an oil-liquid system, a gel system, a powder dispersion system, a water-oil two-layer system, or a water-oil-powder three-layer system.

Besides, it has been known that multiple steps, including a tyrosinase-related step and transportation to the epidermis, are involved in the whitening effect. The whitening effect can be enhanced by the combined use of various whitening compounds, including the representative compounds having whitening activity compounds described in the Background Art.

The external preparation for skin according to the embodiment preferably has a pH of 0.5 to 12, more preferably 3 to 7, and particularly preferably 3.5 to 7. By setting the pH within such a range, the melanogenesis-inhibiting effect of the compound of the formula (I) or a salt thereof is more effectively exerted. Moreover, melanogenesis can be suppressed without destroying normal bacteria in the skin, including melanoma cells.

The melanogenesis inhibitor according to the embodiment can also be mixed with one type or two or more types of commonly used fragrance ingredients to prepare a fragrance composition.

The "commonly used fragrance ingredients" referred to herein include various types of synthetic fragrances, natural essential oils, synthetic essential oils, citrus oils, animal fragrances, and the like. For example, a wide variety of fragrance ingredients can be used, such as those described in "Perfume and Flavor Chemicals (Aroma Chemicals) 1,2" (Steffen Arctender (1969)), "Synthetic Fragrances: Chemistry and Product Knowledge (Expanded and Revised Edition)" by Genichi Indo, The Chemical Daily Co., Ltd., March 22, 2005 (Expanded and Revised Edition), and "Collection of Well-Known and Commonly Used Techniques (Fragrances) Part I" (January 29, 1999, published by the Japan Patent Office). Among others, representative examples of the fragrance ingredients may include α-pinene, limonene, cis-3-hexenol, phenylethyl alcohol, styrallyl acetate, ammonium isovalerate, eugenol, rose oxide, linalool, benzaldehyde, muscone, Musk T (registered trademark, manufactured by Takasago International Corporation), Thesaron (registered trademark, manufactured by Takasago International Corporation), and fragrances having a cooling effect, such as derivatives having a menthol or menthane skeleton. By using these fragrances in combination with the melanogenesis inhibitor according to the embodiment, it becomes possible to improve the fragrance quality and fragrance tone of the blended fragrance.

The melanogenesis inhibitor according to the embodiment does not only have the melanogenesis-inhibiting effect as mentioned above, but may also have one or more of the following effects (a) to (d):
(a) solubility in oils,
(b) solubility in oils and fats,
(c) chemical peeling ability, and
(d) ability to eliminate acne bacteria.

By having both of the effects (a) and/or (b), the melanogenesis inhibitor according to the embodiment has the effect of dissolving keratin plugs and excess sebum, can bring about a melanogenesis-inhibiting effect up to the deep layer of the skin, and is also useful in improving rough skin and acne.

By also having the effect (c), the melanogenesis inhibitor according to the embodiment has the effect of removing unnecessary stratum corneum, can bring about a melanogenesis-inhibiting effect up to the deep layer of the skin, and is also useful in improving the blemishes, dullness, fine wrinkles, and acne of the skin.

By also having the effect (d), the melanogenesis inhibitor according to the embodiment has the effect of removing acne bacteria, which cause acne, and is also useful in improving acne.

Herein, the term "oil" refers to fatty acid that is a liquid at normal temperature, such as, for example, oleic acid, linoleic acid, and linolenic acid. The term "fat and oil" refers to fatty acid, fatty acid ester, or a mixture thereof that is a solid at normal temperature, and examples of the fat and oil may include palmitic acid, lauric acid, myristic acid, stearic acid, palmitic acid glyceride, lauric acid glyceride, myristic acid glyceride, and stearic acid glyceride.

The melanogenesis inhibitor according to the embodiment preferably has any two or more of the above-described effects (a) to (d), from the viewpoint of improving acne and bringing about a melanin-inhibiting effect up to the deep layer of the skin. Moreover, the melanogenesis inhibitor according to the embodiment more preferably has any three or more of the above-described effects, from the viewpoint of improving rough skin and acne and bringing about a melanin-inhibiting effect up to the deep layer of the skin. The melanogenesis inhibitor according to the embodiment further preferably has all of the above-described effects, from the viewpoint of improving the blemishes, dullness, fine wrinkles, rough skin and acne of the skin, and bringing about a melanin-inhibiting effect up to the deep layer of the skin.

### Examples

Hereinafter, the present invention will be specifically described in the following examples. However, these examples are not intended to limit the present invention at all. In addition, various changes or modifications may be added to the present invention without deviating from the scope of the present invention. It is to be noted that, in the formulations below, unless otherwise specified, the unit "%" means "% by weight" and the composition ratio means a weight ratio. The used reagents are those manufactured by FUJIFILM Wako Pure Chemical Corporation, unless otherwise noted. Methyl lactic acid used was manufactured by Mitsubishi Gas Chemical Company, Inc.

### < Example 1: Evaluation of melanin production inhibitory activity >

### - Cell culture conditions

As melanin-producing cells, mouse-derived skin melanoma cells, B16 melanoma (JCRB0202, purchased from JCRB Cell Bank) were used. As a medium for cell culture, 500 mL of E-MEM medium was mixed with 5 mL of x 100 penicillin-streptomycin solution and 100 mL of inactivated fetal bovine serum (manufactured by West Bio), and the resulting medium was used. For the subculture of cells, the cells were washed with a phosphate buffer (manufactured by GIBCO), and were detached from the vessel with 0.25% trypsin and EDTA (ethylenediaminetetraacetic acid), followed by addition of a medium, and the obtained mixture was centrifugated to separate the cels. To ensure stable cell culture, the cell concentration was adjusted to 5 to 16 x 10⁴ cells/mL, and the cells were then cultured in a 10-cm petri dish or a plastic culture flask (175 cm²) in the presence of 5% carbon dioxide at 37°C.

As an assay medium, 500 mL of D-MEM (phenol red-free, glutamic acid-free) was mixed with 5 mL of x 100 penicillin-streptomycin solution, 100 mL of inactivated fetal bovine serum (manufactured by West Bio), and 5 mL of GlutaMax x 100 (manufactured by GIBCO), and the obtained mixture was used.

### - Test Sample

As a test sample (100 µM methyl lactic acid), NaOH was added to methyl lactic acid to adjust the pH to 6.5, and was used. The methyl lactic acid was added into the assay medium to result in a concentration of 100 µM.

**[Table 1]**

| Table 1: Composition ratio of test sample | | |
|---|---|---|
| Reagent | Amount added | Concentration |
| Methyl lactic acid | 10.4mg | 1.04 x 10⁻³ % by weight (100 µM) |
| NaOH | 3.8mg | 0.38 x 10⁻³ % by weight (95 µM) |
| Assay medium | 1000g | 99.999% by weight |

### - Conditions for measuring melanin production percentage

Cells were detached from the culture obtained under the aforementioned cell culture conditions, and 1 mL of cells and medium were each seeded into a 12-well vessel to a cell concentration of 40 x 10⁴ cells/mL. After one day of culture, the medium was removed and replaced with the test sample. After one day of culture, the vessel was washed with the assay medium, and 125 µL of 1M NaOH was added to lyse the cells. 100 µL of the lysed cell lysate was placed in a 96-well vessel. The absorbance at 450 nm was measured using a plate reader, and the melanin concentration was calculated.

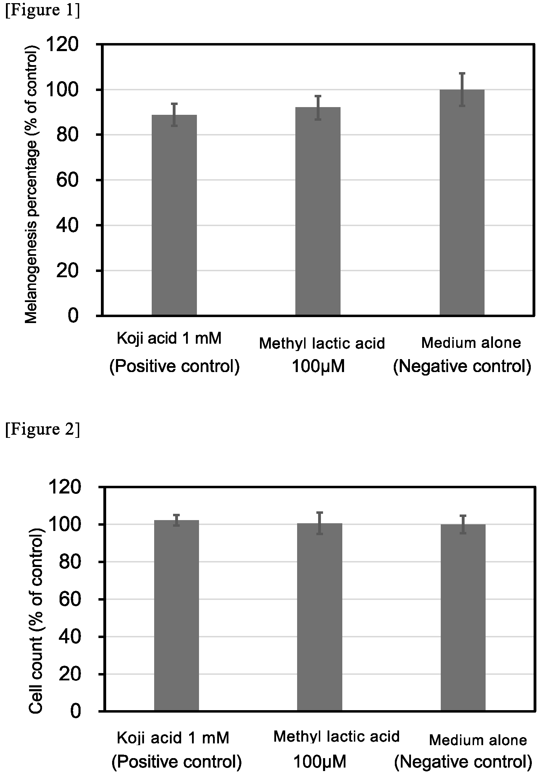

The results are shown in Figure 1. In Figure 1, "Medium alone" is a negative control, which uses an assay medium without the test sample. "Kojic acid 1 mM" is a positive control, which uses an assay medium supplemented with 1 mM kojic acid known to have a melanogenesis-inhibiting effect.

The melanin production percentage (%) shown in Figure 1 is a relative value (%) in a case where the melanin concentration in the negative control is taken as 100%, and for each case, it is an average value of the values measured for 12 samples.

As can be seen from Figure 1, the addition of methyl lactic acid had the same melanogenesis-inhibiting effect as the addition of kojic acid.

### < Example 2: Cytotoxicity test >

### - Cell culture conditions

Cell culture was performed by the same method as that in Example 1 mentioned above.

### - Test sample

The test sample was prepared by the same method as that in Example 1 mentioned above.

### - Cytotoxicity measurement conditions

Cells were detached from the culture obtained under the cell culture conditions described in Example 1, and 0.1 mL of cells and medium were each seeded into a 96-well vessel to a cell concentration of 5 x 10⁴ cells/mL. After one day of culture, the medium was removed and replaced with the test sample. After one further day of culture, the vessel was washed with an assay medium, and 0.1 mL each of a 1 : 9 mixed solution of Cell counting Kit 8 and medium was added. After the mixture had been left at rest at 37°C for 30 minutes in the presence of 5% carbon dioxide, the absorbance at 450 nm was measured using a plate reader, and the cell count was calculated.

The results are shown in Figure 2. The cell count (%) is a relative value (%) in a case where the cell count in the negative control (medium alone) is taken as 100%, and for each case, it is an average value of the values measured for 16 samples.

As can be seen in Figure 2, cytotoxicity was not confirmed when methyl lactic acid was added.

### < Example 3: Tyrosinase biosynthesis inhibitory action test >

### - Cell culture conditions

Normal human melanocytes (manufactured by Kurabo Industries, Ltd.) were used as melanin-producing cells. As a medium for the cells, Dermalife (manufactured by Kurabo Industries, Ltd.) was used. The cell concentration was adjusted to 3 x 10⁴ cells/mL, and the cells were cultured in a 96-well plate in the presence of 5% carbon dioxide at 37°C for 24 hours.

### - Test sample

As a test sample, NaOH was added to methyl lactic acid to adjust the pH to 6.5, and was used. The test sample was added into Dermalife, so that methyl lactic acid had each of concentrations of 2.5, 5, 10, 20, and 40 mM therein. As a control, Dermalife, into which methyl lactic acid was not added, was used.

### - Conditions for measuring tyrosinase biosynthesis inhibitory action

The medium was removed from the culture obtained under the aforementioned cell culture conditions and replaced with the test sample. After one day of culture, the medium was removed, and a phosphate buffer containing 0.5% Triton X-100 was added in an amount of 50 µL/well. The cells were lysed by agitation. 50 µL of the cell lysate was mixed with 50 µL of phosphate buffer containing 2 mM DOPA, and the obtained mixture was then incubated at 37°C for 2 hours. The absorbance (measurement wavelength: 405 nm, reference wavelength: 650 nm) was measured, and the amount of DOPA-melanin was calculated from a calibration curve. The amount of the protein was quantified using a BCA protein assay kit (Thermo), and the amount of DOPA-melanin per unit protein was then calculated to confirm tyrosinase biosynthesis inhibitory action.

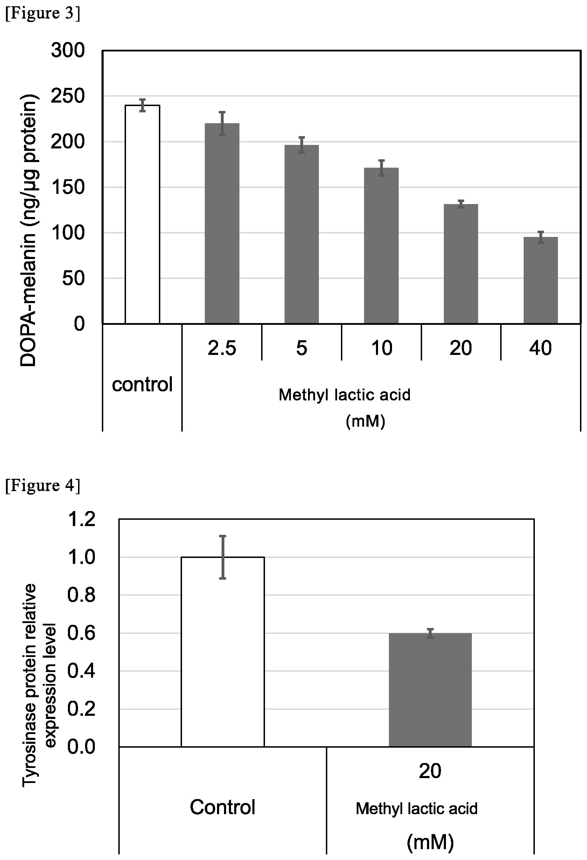

The results are shown in Figure 3. In Figure 3, "Control" is a negative control, which uses a medium without the test sample.

DOPA-melanin (ng/µg protein) shown in Figure 3 is the amount of DOPA-melanin produced per unit protein. If the biosynthesis of tyrosinase is inhibited, the amount of DOPA-melanin will be decreased.

As can be seen from Figure 3, when methyl lactic acid was added, a tyrosinase biosynthesis inhibitory action was exhibited in a dose-dependent manner.

### < Example 4: Tyrosinase protein expression inhibition test >

### - Cell culture conditions

Normal human melanocytes (manufactured by Kurabo Industries Ltd.) were used as melanin-producing cells. As a medium for the cells, Dermalife was used. The cell concentration was adjusted to 3 x 10⁵ cells/1.5 mL, and the cells were cultured in a 6-well plate in the presence of 5% carbon dioxide at 37°C for 24 hours.

### - Test sample

As a test sample, NaOH was added to methyl lactic acid to adjust the pH to 6.5, and was used. The test sample was added into Dermalife, so that methyl lactic acid had a concentration of 20 mM therein. As a control, Dermalife, into which methyl lactic acid was not added, was used.

### - Conditions for measuring tyrosinase protein expression inhibitory action

The medium was replaced with the test sample (1.5 mL/well) and cultured for 48 hours. After removing the medium, proteins were extracted using M-PER^{™} Mammalian Protein Extraction Reagent (manufactured by Themo), and were then developed by SDS-PAGE. After the resultant had been transferred onto a polyvinylidene difluoride (PVDF) membrane, the membrane was blocked with 5% skim milk. After a reaction had been performed with an anti-tyrosinase antibody (manufactured by Santa Cruz Biotechnology) for 1 hour, the membrane was further reacted with an HRP-conjugated secondary antibody (manufactured by abcam) for an additional 1 hour. Protein bands were detected using Ez West Blue W (manufactured by ATTO). The signal intensity of the resulting bands was detected using the image processing software ImageJ, and the relative protein expression levels were calculated.

The results are shown in Figure 4. In Figure 4, "Control" is a negative control, which uses a medium without the test sample.

Figure 4 shows the relative expression level of a tyrosinase protein. If the expression of a tyrosinase protein is inhibited, the relative expression of the tyrosinase protein will be decreased.

As can be seen from Figure 4, when methyl lactic acid was added, a tyrosinase protein expression inhibitory action was exhibited.

### < Example 5: Evaluation of suppression of melanin synthesis-related factor mRNA expression >

### - Cell culture conditions

Normal human melanocytes (manufactured by Kurabo Industries, Ltd.) were used as melanin-producing cells. As a medium for the cells, Dermalife was used. The cell concentration was adjusted to 3 x 10⁴ cells/mL, and the cells were cultured in a 96-well plate in the presence of 5% carbon dioxide at 37°C for 24 hours.

### - Test sample

As a test sample, NaOH was added to methyl lactic acid to adjust the pH to 6.5, and was used. The test sample was added into Dermalife, so that methyl lactic acid had each of concentrations of 10 and 20 mM therein. As a control, Dermalife, into which methyl lactic acid was not added, was used.

### - Conditions for measuring melanin synthesis-related factor mRNA expression-suppressing action

The medium was replaced with the test sample (100 µL/well) and cultured for 24 hours. After removing the medium, RNA was extracted using Cells-to-Ct kit (manufactured by Themo), and was then subjected to reverse transcription to synthesize cDNA. Using the resulting cDNA, Real-time PCR was carried out, and the relative expression level of mRNA was calculated.

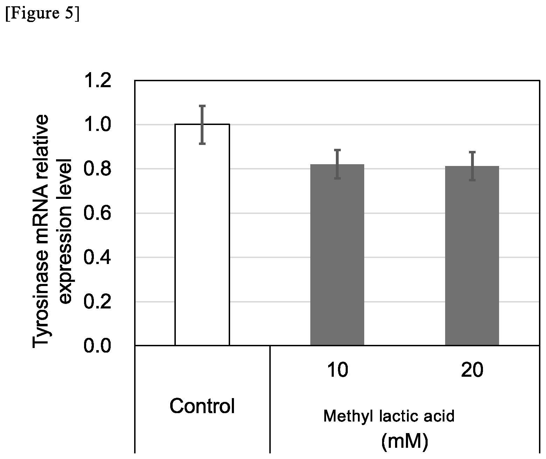

The results are shown in Figure 5. In Figure 5, "Control" is a negative control, which uses a medium without the test sample.

Figure 5 shows the relative expression level of tyrosinase mRNA. When methyl lactic acid was added, it exhibited a tyrosinase mRNA expression-suppressing action.

Formulation examples are shown below. The methyl lactic acid used in the following formulation examples is the same as that used in Example 1.

### < Formulation Example 1 > Whitening wipe-off lotion

The ingredients shown in the following Table 2 were dissolved while stirring at room temperature to prepare a whitening wipe-off lotion.

**[Table 2]**

| No. | Whitening wipe-off lotion (Ingredient names) | % by weight |
|---|---|---|
| 1 | Methyl lactic acid | 5.00 |
| 2 | 1,3-Butylene glycol | 5.70 |
| 3 | Sodium pyrrolidonate | 0.48 |
| 4 | Allantoin | 0.10 |
| 5 | Trehalose | 0.03 |
| 6 | Ethanol | 5.74 |
| 7 | Sucrose laurate | 0.30 |
| 8 | Peg-50 hydrogenated castor oil | 0.19 |
| 9 | Antiseptic | Appropriate amount |
| 10 | pH adjuster | Appropriate amount |
| 11 | Antioxidant | Appropriate amount |
| 12 | Water | Balance |

### < Formulation Example 2 > Whitening lotion

The ingredients shown in the following Table 3 were dissolved while stirring at room temperature to prepare a whitening lotion.

**[Table 3]**

| No. | Whitening lotion (Ingredient names) | % by weight |
|---|---|---|
| 1 | Methyl lactic acid | 5.00 |
| 2 | 1,3-Butylene glycol | 4.77 |
| 3 | Trehalose | 0.03 |
| 4 | Sodium hyaluronate | 0.01 |
| 5 | Di(cholesteryl/behenyl/octyldodecyl) lauroyl glutamate | 0.48 |
| 6 | Triethylhexanoin | 0.29 |
| 7 | Peg-50 hydrogenated castor oil triisostearate | 0.86 |
| 8 | Ceteth-20 | 0.22 |
| 9 | Peg-60 hydrogenated castor oil | 0.84 |
| 10 | Antiseptic | Appropriate amount |
| 11 | pH adjuster | Appropriate amount |
| 12 | Antioxidant | Appropriate amount |
| 13 | Water | Balance |

### < Formulation Example 3 > Whitening cream

The ingredients shown in the following Table 4 were dissolved while stirring at room temperature to prepare a whitening cream.

**[Table 4]**

| No. | Whitening cream (Ingredient names) | % by weight |
|---|---|---|
| 1 | Methyl lactic acid | 5.00 |
| 2 | Mineral oil | 5.94 |
| 3 | Myristyl myristate | 3.80 |
| 4 | Behenyl alcohol | 3.04 |
| 5 | Dimethicone | 2.85 |
| 6 | Di(octyldodecyl/phytosteryl/behenyl) lauroyl glutamate | 1.90 |
| 7 | Dipentaerythrityl tri-polyhydroxystearate | 1.43 |
| 8 | Polyglyceryl-2 triisostearate | 0.95 |
| 9 | Triethylhexanoin | 0.71 |
| 10 | Macadamia ternifolia seed oil | 0.48 |
| 11 | Glycerin | 7.60 |
| 12 | 1,3-Butylene glycol | 5.06 |
| 13 | Peg-75 | 0.95 |
| 14 | Trehalose | 0.48 |
| 15 | Sodium hyaluronate | 0.10 |
| 16 | Glyceryl stearate | 1.90 |
| 17 | Peg-40 stearate | 0.95 |
| 18 | Disodium EDTA | 0.10 |
| 19 | Xanthan gum | 0.10 |
| 20 | Antiseptic | Appropriate amount |
| 21 | pH adjuster | Appropriate amount |
| 22 | Antioxidant | Appropriate amount |
| 23 | Water | Balance |

### < Formulation Example 4 > Whitening emulsion

The ingredients shown in the following Table 5 were dissolved while stirring at room temperature to prepare a whitening emulsion.

**[Table 5]**

| No. | Whitening emulsion (Ingredient names) | % by weight |
|---|---|---|
| 1 | Methyl lactic acid | 5.00 |
| 2 | Mineral oil | 9.50 |
| 3 | Cetyl ethylhexanoate | 1.90 |
| 4 | Beeswax | 0.95 |
| 5 | Phytosteryl/octyldodecyl lauroyl glutamate | 0.95 |
| 6 | Cetanol | 0.48 |
| 7 | Glycerin | 4.75 |
| 8 | 1,3-Butylene glycol | 2.85 |
| 9 | Sodium hyaluronate | 0.03 |
| 10 | Trehalose | 0.03 |
| 11 | Glyceryl stearate | 0.95 |
| 12 | Ceteth-20 | 0.90 |
| 13 | Sorbeth-30 tetraoleate | 0.10 |
| 14 | Peg-45 stearate | 0.05 |
| 15 | Carbomer | 0.12 |
| 16 | Xanthan gum | 0.12 |
| 17 | Disodium EDTA | 0.02 |
| 18 | Antiseptic | Appropriate amount |
| 19 | pH adjuster | Appropriate amount |
| 20 | Antioxidant | Appropriate amount |
| 21 | Water | Balance |

### < Formulation Example 5 > Whitening gel

The ingredients shown in the following Table 6 were dissolved while stirring at room temperature to prepare a whitening gel.

**[Table 6]**

| No. | Whitening gel (Ingredient names) | % by weight |
|---|---|---|
| 1 | Methyl lactic acid | 5.00 |
| 2 | Glycerin | 10.56 |
| 3 | 1,3-Butylene glycol | 5.94 |
| 4 | Trehalose | 0.22 |
| 5 | Sodium hyaluronate | 0.05 |
| 6 | Hydrolyzed hyaluronic acid | 0.01 |
| 7 | Triethylhexanoin | 1.33 |
| 8 | Diphenyl dimethicone | 0.95 |
| 9 | Arginine | 0.67 |
| 10 | Peg-60 hydrogenated castor oil | 0.57 |
| 11 | Carbomer | 0.48 |
| 12 | Polyglyceryl-10 myristate | 0.30 |
| 13 | Disodium EDTA | 0.01 |
| 14 | Antiseptic | Appropriate amount |
| 15 | pH adjuster | Appropriate amount |
| 16 | Water | Balance |

### < Formulation Example 6 > Whitening serum

The ingredients shown in the following Table 7 were dissolved while stirring at room temperature to prepare a whitening serum.

**[Table 7]**

| No. | Whitening serum (Ingredient names) | % by weight |
|---|---|---|
| 1 | Methyl lactic acid | 5.00 |
| 2 | Liquid paraffin | 9.50 |
| 3 | Cetyl ethylhexanoate | 1.90 |
| 4 | White beeswax | 0.95 |
| 5 | Cetanol | 0.48 |
| 6 | Concentrated glycerin | 4.75 |
| 7 | 1,3-Butylene glycol | 2.85 |
| 8 | Sodium hyaluronate (2) | 0.03 |
| 9 | Lipophilic glyceryl monostearate | 0.95 |
| 10 | Polyoxyethylene cetyl ether | 0.90 |
| 11 | Polyoxyethylene sorbitol tetraoleate | 0.10 |
| 12 | Polyethylene glycol monostearate | 0.05 |
| 13 | Carboxyvinyl polymer | 0.12 |
| 14 | Xanthan gum | 0.12 |
| 15 | Disodium EDTA | 0.02 |
| 16 | Antiseptic | Appropriate amount |
| 17 | pH adjuster | Appropriate amount |
| 18 | Antioxidant | Appropriate amount |
| 19 | Purified water | Balance |

### < Formulation Example 7 > Whitening facial cleanser

The ingredients shown in the following Table 8 were dissolved while stirring at room temperature to prepare a whitening facial cleanser.

**[Table 8]**

| No. | Raw materials | Composition ratio |
|---|---|---|
| 1 | Methyl lactic acid | 5.00 |
| 2 | Myristic acid | 13.71 |
| 3 | Stearic acid | 4.57 |
| 4 | Palmitic acid | 3.66 |
| 5 | Lauric acid | 3.66 |
| 6 | Glycerin | 14.62 |
| 7 | 1,3-Butylene glycol | 4.57 |
| 8 | Sodium lauroyl sarcosinate | 2.75 |
| 9 | Lauryl betaine | 1.28 |
| 10 | Sodium lauroyl methylalanine | 1.09 |
| 11 | Hydrogenated castor oil | 0.91 |
| 12 | Behenyl alcohol | 0.46 |
| 13 | Tocopheryl acetate | 0.18 |
| 14 | Polyquaternium-7 | 0.09 |
| 15 | Potassium hydroxide | 5.94 |
| 16 | Glyceryl stearate (SE) | 1.82 |
| 17 | Glycol distearate | 1.82 |
| 18 | Poloxamer 215 | 0.46 |
| 19 | Antiseptic | Appropriate amount |
| 20 | pH adjuster | Appropriate amount |
| 21 | Chelating agent | Appropriate amount |
| 22 | Water | Balance |

### < Formulation Example 8 > Whitening soap

The ingredients shown in the following Table 9 were dissolved while stirring at 80°C in a hot-water bath, and were then cooled and solidified to prepare a whitening soap.

**[Table 9]**

| No. | Raw materials | Composition ratio |
|---|---|---|
| 1 | Methyl lactic acid | 5.00 |
| 2 | Sodium myristate | 11.88 |
| 3 | Sodium laurate | 6.18 |
| 4 | Sodium palmitate | 6.18 |
| 5 | Sodium stearate | 5.70 |
| 6 | Potassium myristate | 4.75 |
| 7 | Potassium laurate | 2.85 |
| 8 | Potassium palmitate | 2.85 |
| 9 | Sodium isostearate | 2.85 |
| 10 | Potassium stearate | 2.85 |
| 11 | Sodium castor fatty acid | 2.38 |
| 12 | Potassium isostearate | 0.71 |
| 13 | Potassium castor oil fatty acid | 0.67 |
| 14 | Sorbitol | 11.88 |
| 15 | Sucrose | 11.88 |
| 16 | 1,3-Butylene glycol | 4.75 |
| 17 | Diglycerin | 2.85 |
| 18 | Glycerin | 0.29 |
| 19 | Sodium chloride | 0.67 |
| 20 | Chelating agent | Appropriate amount |
| 21 | Ph adjuster | Appropriate amount |
| 22 | Water | Balance |

### < Example 6 and Comparative Examples 1 to 3: Tests regarding other effects >

### (Solubility in oils)

Oleic acid was added into an aqueous solution of 50% by weight of organic acid, and the mixed solution was then stirred at room temperature (23°C) for 24 hours. At that time, if the saturated solubility of oleic acid was 200 ppm or more, it was evaluated as A; if it was less than 200 ppm, it was evaluated as B.

### (Solubility in oils and fats)

Palmitic acid was added into an aqueous solution of 50% by weight of organic acid, and the mixed solution was then stirred at room temperature (23°C) for 24 hours. At that time, if the saturated solubility of palmitic acid was 20 ppm or more, it was evaluated as A; if it was less than 20 ppm, it was evaluated as B.

### (Chemical peeling ability)

An aqueous solution of 7% by weight of organic acid was allowed to act on to pig skin for 24 hours. If the thickness of the stratum corneum decreased by 10% or more, it was evaluated as A; if it decreased by less than 10%, it was evaluated as B.

### (Ability to eliminate acne bacteria)

0.05 mL of an aqueous solution of 7% by weight of organic acid was added dropwise to 5 mL of a test solution containing the bacterial strain *Cutibacterium acnes* NBRC 107605 at a concentration of 108 CFU/mL. After 60 seconds, if the viable cell count was less than 1%, it was evaluated as A; if the viable cell count was 1% or more, it was evaluated as B.

**[Table 10]**

| | Main ingredient (Organic acid) | Ability to suppress melanogenesis | Solubility in oils | Solubility in oils and fats | Chemical peeling ability | Ability to eliminate acne bacteria |
|---|---|---|---|---|---|---|
| Example 6 | Methyl lactic acid | A | A | A | A | A |
| Comp. Ex. 1 | Glvcolic acid | | B | B | A | A |
| Comp. Ex. 2 | Lactic acid | | B | B | A | A |
| Comp. Ex. 3 | Kojic acid | A | B | B | B | B |

From the above-described test results, it is found that methyl lactic acid has all of the solubility in oils, the solubility in oils and fats, the chemical peeling ability, and the ability to eliminate acne bacteria, in addition to the melanogenesis-inhibiting effect.

Several embodiments of the present invention have been described. These embodiments are presented as examples and are not intended to limit the scope of the invention. These novel embodiments can be carried out in various other forms, and various omissions, replacements and modifications can be made in a range that does not depart from the gist of the invention. These embodiments and variations thereof are included in the scope and gist of the invention, and are also included in the scope of the inventions recited in the claims and the equivalents thereof.

## Claims

1. A melanogenesis inhibitor, comprising a compound represented by the following formula (I) or a salt thereof: 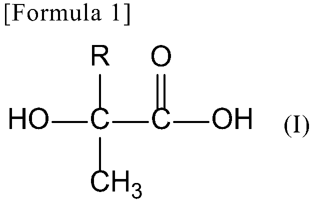 wherein R represents an alkyl group containing 1 to 4 carbon atoms.

2. The melanogenesis inhibitor according to claim 1, wherein R in the formula (I) is a methyl group.

3. The melanogenesis inhibitor according to claim 1 or 2, which further has any one or more of the following effects (a) to (d):
(a) solubility in oils,
(b) solubility in oils and fats,
(c) chemical peeling ability, and
(d) ability to eliminate acne bacteria.

4. An external preparation for skin, comprising the melanogenesis inhibitor according to any one of claims 1 to 3.

5. The external preparation for skin according to claim 4, which comprises the compound represented by the formula (I) or a salt thereof in an amount of 0.001% to 50% by weight with respect to the external preparation for skin.

6. The external preparation for skin according to claim 4 or 5, which further comprises one or more additives selected from the group consisting of a thickener, a pH adjuster, an antiseptic, and an antioxidant.

7. The external preparation for skin according to claim 6, which comprises the additive(s) in each amount of 0.01% to 20% by weight each.

8. The external preparation for skin according to any one of claims 4 to 7, which has a pH value of 0.5 to 12.

9. The external preparation for skin according to any one of claims 4 to 8, which is a whitening cosmetic.

10. The external preparation for skin according to claim 9, wherein the whitening cosmetic is selected from the group consisting of the group consisting of a lotion, a cream, an emulsion, a gel, a serum, a facial cleanser, a soap, an ointment, a pack agent, and a foundation.
